# EUROPÄISCHE PATENTSCHRIFT

(11) **EP 0 544 168 B1**
(45) Veröffentlichungstag und Bekanntmachung des Hinweises auf die Patenterteilung: **03.09.1997**
(21) Anmeldenummer: 92119530.1
(22) Anmeldetag: 16.11.1992
(51) Int. Cl.: C07D 223/20, C07D 413/12, A61K 31/55

(54) **Verwendung von Aminoalkyl-substituierten 5,6-Dihydro-dibenz(b,e)azepin-6,11-dion-11-oximen als antivirale Mittel**
Use of aminoalkyl substituted 5,6-dihydro-dibenzo(b,e)azepin-6,11-dion-11-oximes as antiviral agents
Utilisation de 5,6-dihydro-dibenzo(b,e)azépin-6,11-dion-11-oximes substituées par un aminoalkyl en tant qu'agents antiviraux

(30) Priorität: 27.11.1991 DE 4138909
(43) Veröffentlichungstag der Anmeldung: 02.06.1993
(73) Patentinhaber: BAYER AG, 51368 Leverkusen (DE)
(72) Erfinder: Wild, Hanno, Dr., W-5600 Wuppertal 1 (DE); Roeben, Wolfgang, Dr., W-5060 Bergisch Gladbach (DE); Aichinger, Gerd, Dr., W-5600 Wuppertal 1 (DE); Paessens, Arnold, Dr., W-5657 Haan (DE); Petersen-von Gehr, Jörg, Dr., W-4630 Bochum 1 (DE)

(56) Entgegenhaltungen:
- FR-M- 6 753
- GB-A- 1 132 516
- JOURNAL OF MEDICINAL CHEMISTRY Bd. 34, Nr. 7, Juli 1991, WASHINGTON US Seiten 2231-2241 K.D. HARGRAVE ET AL.

## Beschreibung

Die Erfindung betrifft die Verwendung von Aminoalkyl-substituierten 5,6-Dihydro-dibenz[b,e]azepin-6,11-dion-11-oximen.

Aus der DE 15 45 856 ist ein Verfahren zur Herstellugn von basisch substituierten Derivaten des 5,6-dihydro-dibenz[b,e]azepin-6,11-dion-11-oxims bekannt, wobei dort auch einige wenige Beispiele mit Aminoalkylresten am Oximsauerstoff beschrieben sind.

Ferner sind aus dem US-Patent 34 31 257 einige basisch substituierte 5,6-Dihydro-dibenz[b,e]azepin-6,11-dion-11-oxime mit psychotropischer Wirkung bekannt, wobei die erfindungsgemäßen Verbindungen der allgemeinen Formel (I) teilweise vom Wortlaut des Bedeutungsumfanges dieser Publikationen erfaßt werden.

Die vorliegende Erfindung betrifft jetzt die Verwendung von Aminoalkyl-substitutierten 5,6-Dihydro-dibenz[b,e]azepin-6,11-dion-11-oximen der allgemeinen Formel (I) 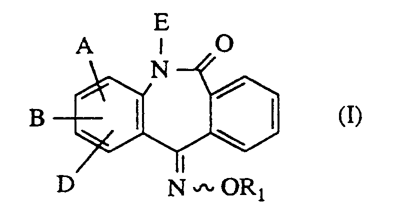 in welcher
- A, B und D: gleich oder verschieden sind und für Wasserstoff, Amino, Nitro, Halogen, Cyano, Hydroxy, Trifluormethyl, Trifluormethoxy oder für geradkettiges oder verzweigtes Alkyl oder Alkoxy mit jeweils bis zu 8 Kohlenstoffatomen stehen,
- E: für Wasserstoff oder für geradkettiges oder verzweigtes Alkyl mit bis zu 6 Kohlenstoffatomen steht,
- R¹: für geradkettiges oder verzweigtes Alkyl oder Alkenyl mit jeweils bis zu 10 Kohlenstoffatomen steht, die stets durch eine Gruppe der Formel -NR²R³ substituiert sind,
worin
- R² und R³: gleich oder verschieden sind und Wasserstoff, geradkettiges oder verzweigtes Alkyl mit bis zu 8 Kohlenstoffatomen oder Phenyl bedeuten, oder
- R² und R³: gemeinsam mit dem Stickstoffatom einen 5- bis 7-gliedrigen, gesättigten oder ungesättigten heterocyclischen Ring mit bis zu 2 weiteren Heteroatomen aus der Reihe S, N oder O bilden,
gegebenenfalls in einer isomeren Form und deren physiologisch unbedenklichen Salze zur Herstellung von Arzneimitteln zur Therapie von retroviralen Infektionen.

Physiologisch unbedenkliche Salze der Aminoalkyl-substituierten 5,6-Dihydro-dibenz[b,e]azepin-6,11-dion-11-oxime können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Heterocyclus steht im allgemeinen für eine 5- bis 7-gliedrigen, bevorzugt 5- bis 6-gliedrigen, gesättigten oder ungesättigten Ring, der als Heteroatome bis zu 2 Sauerstoff-, Schwefel- und/oder Stickstoffatome enthalten kann. Bevorzugt sind 5- und 6-gliedrige Ringe mit einem Sauerstoff-, Schwefel- und/oder bis zu 2 Stickstoffatomen. Besonders bevorzugt werden genannt: Pyrrolyl, Pyrazolyl, Pyridyl, Pyrimidyl, Pyrazinyl, Pyridazinyl, Thiazolyl, Oxazolyl, Imidazolyl, Isoxazolyl, Pyrrolidinyl, Piperidinyl, Piperazinyl, Tetrazolyl oder Morpholinyl.

Die erfindungsgemäß verwendbaren Verbindungen können in stereoisomeren Formen existieren, die sich entweder wie Bild und Spiegelbild (Enantiomere), oder die sich nicht wie Bild und Spiegelbild (Diastereomere) verhalten. Gemeint sind sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen (vgl. E. L. Eliel, Stereochemistry of Carbon Compounds, McGraw Hill, 1962).

Bei dem Rest der allgemeinen Formel (II) 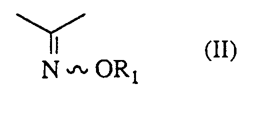 kann die C=N-Doppelbindung sowohl die E- als auch die Z-Konfiguration besitzen bzw. es können E/Z-Gemische vorliegen.

Bevorzugt ist die Verwendung von Verbindungen der allgemeinen Formel (I), in welcher
- A, B und D: gleich oder verschieden sind und für Wasserstoff, Fluor, Chlor, Hydroxy, Trifluormethyl, Trifluormethoxy oder für geradkettiges oder verzweigtes Alkyl oder Alkoxy mit jeweils bis zu 6 Kohlenstoffatomen stehen,
- E: für Wasserstoff oder für geradkettiges oder verzweigtes Alkyl mit bis zu 4 Kohlenstoffatomen steht,
- R¹: für geradkettiges oder verzweigtes Alkyl oder Alkenyl mit jeweils bis zu 8 Kohlenstoffatomen steht, die stets durch eine Gruppe der Formel -NR²R³ substituiert sind,
worin
- R² und R³: gleich oder verschieden sind und Wasserstoff oder geradkettiges oder verzweigtes Alkyl mit bis zu 6 Kohlenstoffatomen bedeuten,
oder
- R² und R³: gemeinsam mit dem Stickstoffatom einen Morpholin- oder Piperazinring bilden,
gegebenenfalls in einer isomeren Form und deren physiologisch unbedenklichen Salze, zur Herstellung von antiviral wirksamen Verbindungen.

Besonders bevorzugt ist die Verwendung von Verbindungen der allgemeinen Formel (I), in welcher
- A, B und D: gleich oder verschieden sind und für Wasserstoff, Fluor, Chlor oder für geradkettiges oder verzweigtes Alkyl oder Alkoxy mit jeweils bis zu 4 Kohlenstoffatomen stehen,
- E: für Wasserstoff, Methyl oder Ethyl steht,
- R¹: für geradkettiges oder verzweigtes Alkyl oder Alkenyl mit jeweils bis zu 6 Kohlenstoffatomen steht, die stets durch eine Gruppe der Formel -NR²R³ substituiert sind,
worin
- R² und R³: gleich oder verschieden sind und Wasserstoff oder geradkettiges oder verzweigtes Alkyl mit bis zu 4 Kohlenstoffatomen bedeuten,
oder
- R² und R³: gemeinsam mit dem Stickstoffatom einen Morpholinring bilden,
gegebenenfalls in einer isomeren Form und deren physiologisch unbedenklichen Salze zur Herstellung von antiviral wirksamen Arzneimitteln.

Die Verbindungen der allgemeinen Formel (I) können hergestellt werden, indem man
[A] Verbindungen der allgemeinen Formel (III) 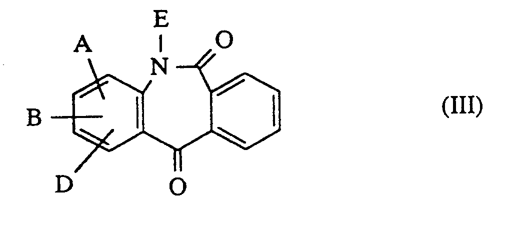 in welcher
   - A, B, D und E: die oben angegebene Bedeutung haben,
   mit Hydroxylaminen der allgemeinen Formel (IV)

   H₂N-OR¹ (IV)

   in welcher
   - R¹: die oben angegebene Bedeutung hat,
   in inerten Lösemitteln, gegebenenfalls in Anwesenheit einer Base umsetzt,
   oder
[B] Verbindungen der allgemeinen Formel (V) 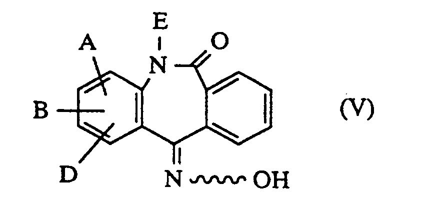 in welcher
   - A, B, D und E: die oben angegebene Bedeutung haben,
   entweder mit Verbindungen der allgemeinen Formel (VI)

   L-R¹ (VI)

   in welcher
   - R¹: die oben angegebene Bedeutung hat
   und
   - L: für eine typische Abgangsgruppe, wie beispielsweise Tosylat, Mesylat, Chlor, Brom oder Jod steht,
   oder im Fall, daß R² und R³ Wasserstoff bedeuten, mit Verbindungen der allgemeinen Formel (VII)

   L-R⁴-T (VII)

   in welcher
   - L: die oben angegebene Bedeutung hat
   - R⁴: für (C₁-C₈)-Alkyl steht
   und
   - T: für Phthalimido steht,
   ebenfalls in inerten Lösemitteln in Anwesenheit einer Base umsetzt,
   und anschließend mit Hydrazinhydrat umsetzt,
   und gegebenenfalls die Substituenten A, B, D und R¹ nach üblichen chemischen Methoden variiert,
   und im Fall, daß E nicht Wasserstoff bedeutet, ebenfalls nach bekannten Methoden eine Alkylierung durchführt

Die Verfahren können durch folgendes Formelschema bespielhaft erläutert werden:
[A] 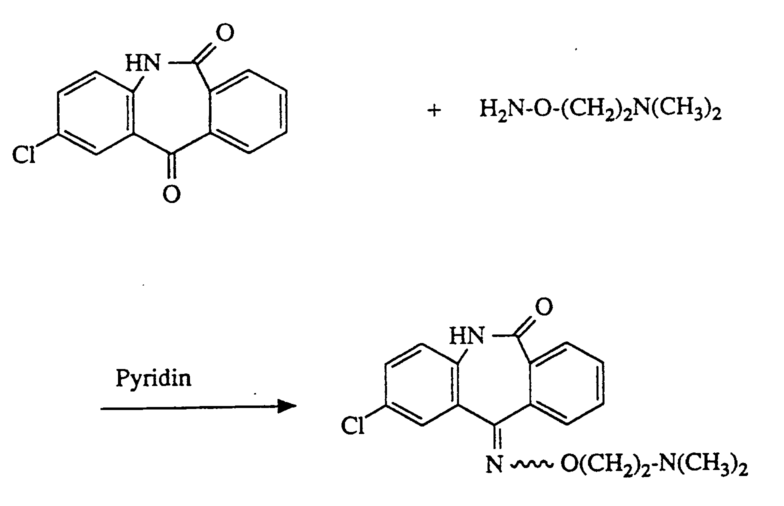
[B]

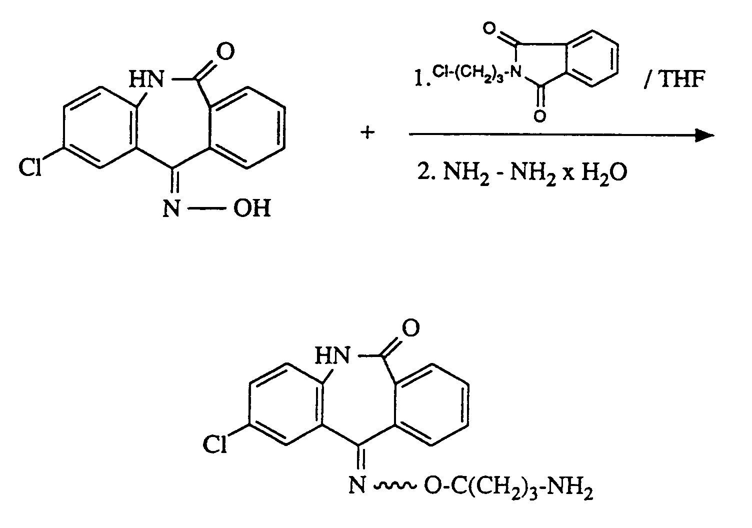

Die oben aufgeführten Verfahren erfolgen in Analogie zu den beschriebenen Methoden aus dem US-Patent 34 31 257.

Als Lösemittel eignen sich für die Verfahren [A] und [B] die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind Pyridin und Tetrahydrofuran.

Als Basen eignen sich die üblichen basischen Verbindungen. Hierzu gehören vorzugsweise Alkali- oder Erdalkalihydroxide, wie Lithiumhydroxid, Natriumhydroxid, Kaliumhydroxid oder Bariumhydroxid, Alkalihydride wie Natriumhydrid, Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.buylat, oder organische Amine wie Benzyltrimethylammoniumhydroxid, Tetrabutylammoniumhydroxid, Pyridin, Triethylamin oder N-Methylpiperidin.

Die Verfahren [A] und [B] werden im allgemeinen in einem Temperaturbereich von +0°C bis +150°C, bevorzugt von +0°C bis +120°C durchgeführt.

Im allgemeinen wird bei Normaldruck gearbeitet. Es ist aber auch möglich, das Verfahren bei Unterdruck oder bei Überdruck durchzuführen (z.B. in einem Bereich von 0,5 bis 5 bar).

Als Lösemittel für die Alkylierung (E ≠ H) eignen sich ebenfalls übliche organische Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Aceton.

Die Alkylierung wird in den oben aufgeführten Lösemitteln bei Temperaturen von 0°C bis +150°C, vorzugsweise bei Raumtemperaturen bis +100°C, bei Normaldruck durchgeführt.

Die Verbindungen der allgemeinen Formel (III) sind an sich bekannt oder können nach üblichen Methoden hergestellt werden [vgl. z.B. US 34 31 257].

Ebenso sind die Hydroxylamine der allgemeinen Formel (IV) bekannt.

Die Verbindungen der allgemeinen Formel (V) werden teilweise oder vom Bedeutungsumfang des US-Patents 34 31 257 (E ≠ H) erfaßt und können dann nach dem dort beschriebenen Verfahren hergestellt werden.

Die Verbindungen der allgemeinen Formeln (VI) und (VII) sind bekannt [vgl. Beilstein 1, 114].

Die hier beschriebenen Inhibitoren sind Inhibitoren der Reversen Transkriptase und sind als solche für alle Zwecke einsetzbar, für die Enzyminhibitoren geeignet sind. Dies ist zum Beispiel der Einsatz in der Diagnostik um die Präzision und Selektivität von Enzymaktivitätsmessungen zu verbessern. Bei der Affinitätschromatographie können sie als Affinitätslabel dienen und in der Forschung kann man sie zur Aufklärung von Reaktionsmechanismen enzymatischer Reaktionen verwenden.

Darüber hinaus wurde überraschend gefunden, daß die erfindungsgemäßen Verbindungen der allgemeinen Formel (I) eine außerordentlich starke Wirkung gegen Retroviren besitzen. Sie zeigen Wirkung in Lentivirus-infizierten Zellkulturen. Dies konnte am Beispiel des HIV-Virus gezeigt werden.

### HIV-Infektion in Zellkultur

Der HIV-Test wurde mit geringen Modifikationen nach der Methode von Pauwels et al. [vgl. Journal of Virological Methods 20, (1988), 309-321] durchgeführt.

Normale menschliche Blutlymphozyten (PBL's) wurden über Ficoll-Hypaque angereichert und im RPMI 1640, 20% fötales Kälberserum mit Phytohaemagglutinin (90 µg/ml) und Interleukin-2 (40 U/ml) stimuliert. Zur Infektion mit dem infektiösen HIV wurden PBL's pelletiert und das Zellpellet wurde anschließend in 1 ml HIV-Virusadsorptionslösung suspendiert und 1 Stunde bei 37°C inkubiert.

Alternativ wurden HIV-suszeptible H9-Zellen anstelle von normalen menschlichen Blutlymphozyten zur Testung der antiviralen Effekte der erfindungsgemäßen Verbindungen eingesetzt.

Die Virusadsorptionslösung wurde zentrifugiert und das infizierte Zellpellet in Wachstumsmedium aufgenommen, so daß 1 x 10⁵ Zellen pro ml eingestellt waren. Die derart infizierten Zellen wurden zu 1 x 10⁴ Zellen/Napf in die Näpfe von 96er Mikrotiterplatten pipettiert.

Die erste vertikale Reihe der Mikrotiterplatte enthielt nur Wachstumsmedium und Zellen, die nicht infiziert, aber ansonsten genauso wie oben beschrieben, behandelt worden waren (Zellkontrolle). Die zweite vertikale Reihe der Mikrotiterplatte erhielt nur HIV-infizierte Zellen (Viruskontrolle) in Wachstumsmedium. Die übrigen Näpfe enthielten die erfindungsgemäßen Verbindungen in unterschiedlichen Konzentrationen, ausgehend von den Näpfen der 3. vertikalen Reihe der Mikrotiterplatte, von der die Prüfsubstanzen in 2er Schritten 2¹⁰fach verdünnt wurden.

Die Testansätze wurden so lange bei 37°C inkubiert, bis in der unbehandelten Viruskontrolle die für das HIV typische Syncytienbildung auftrat (zwischen Tag 3 und 6 nach Infektion), die dann mikroskopisch ausgewertet wurde. In der unbehandelten Viruskontrolle resultierten unter diesen Testbedingungen etwa 20 - 50 Syncytien, währen die unbehandelte Zellkontrolle keine Syncytien aufwies.

Die IC₅₀-Werte wurden als die Konzentration der behandelten und infizierten Zellen ermittelt, bei der 50% (ca. 10 - 20 Syncytien) der virusinduzierten Syncytien durch die Behandlung mit der erfindungsgemäßen Verbindung unterdrückt waren.

Es wurde nun gefunden, daß die erfindungsgemäßen Verbindungen HIV infizierte Zellen vor der virusinduzierten Zellzerstörung schützen.

**Tabelle I**

| Bsp.-Nr. | IC₅₀ (µM) |
|---|---|
| 1 | 0,38 |
| 3 (Vergleich) | 1,5 |
| BIRG 587 [J. Med. Chem. 34 2231,(1991)] | 0,09 |

Die erfindungsgemäßen Verbindungen stellen wertvolle Wirkstoffe zur Behandlung und Prophylaxe von Erkrankungen, hervorgerufen durch Retroviren, in der Human- und Tiermedizin dar.

Als Indikationsgebiete in der Humanmedizin können beispielsweise genannt werden:
1.) Die Behandlung und Prophylaxe von menschlichen Retrovirusinfektionen.
2.) Für die Behandlung oder Prophylaxe von HIV I (Virus der humanen Immundefizienz; früher HTLV III/LAV genannt) und HIV II verursachten Erkrankungen (AIDS) und den damit assoziierten Stadien wie ARC (AIDS related complex) und LAS (Lymphadenopathie-Syndrom) sowie der durch dieses Virus verursachten Immunschwäche und Encephalopathie.
3.) Für die Behandlung oder die Prophylaxe einer HTLV-I oder HTLV-II Infektion.
4.) Für die Behandlung oder die Prophylaxe des AIDS-carrier Zustandes (AIDS-Überträger-Zustand).

Als Indikationen in der Tiermedizin können beispielsweise angeführt werden:

Infektionen mit
a) Maedivisna (bei Schafen und Ziegen)
b) progressivem Pneumonievirus (PPV) (bei Schafen und Ziegen)
c) caprine arthritis encephalitis Virus (bei Schafen und Ziegen)
d) Zwoegerziekte Virus (bei Schafen)
e) infektiösem Virus der Anämie (des Pferdes)
f) Infektionen verursacht durch das Katzenleukämievirus
g) Infektionen verursacht durch das Virus der Katzen-Immundefizienz (FIV)
h) Infektionen verursacht durch das Virus der Affen-Immundefizienz (SIV)

Bevorzugt werden aus dem Indikationsgebiet in der Humanmedizin die oben aufgeführten Punkte 2, 3 und 4.

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) enthalten oder die aus einem oder mehreren Wirkstoffen der Formel (I) bestehen, sowie Verfahren zur Herstellung dieser Zubereitungen.

Die Wirkstoffe der Formel (I) sollen in den oben aufgeführten pharmazeutischen Zubereitungen, vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,1 bis etwa 200, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

### Herstellungsbeispiele

### Beispiel 1

### (E/Z)-2-Chlor-11-(2-dimethylaminoethyloxyimino)-6-oxo-5,6-dihydro-11H-dibenz[b,e]azepin

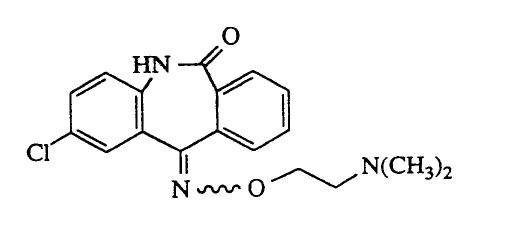

500 mg (2,2 mmol) 2-Chlor-6,11-dioxo-5,6-dihydro-11H-dibenz[b,e]azepin und 428 mg (2,4 mmol) O-[2-(Dimethylamino)ethyl]hydroxylamin-dihydrochlorid in 4,4 ml Pyridin werden 45 h auf 100°C erhitzt. Anschließend wird mit Essigester verdünnt, mit gesättigter Na₂CO₃-Lösung gewaschen und die organische Phase über MgSO₄ getrocknet. Chromatographie an Kieselgel mit Methylenchlorid / Methanol 10:1 liefert 200 mg des Oxims als Schaum.
¹H-NMR (DMSO): δ = 2,30 und 2,32 (2s, 3H); 2,70 (m, 2H); 4,35 (m, 2H); 7,15 und 7,22 (2d, J = 9 Hz, 1H); 7,40 - 7,65 (m, 5H); 7,92 (m, 1H); 10,72 und 10.79 (2s, NH).

### Beispiel 2

### (E/Z)-11-(3-Aminopropyloxyimino)-2-chlor-6-oxo-5,6-dihydro-11H-dibenz[b,e]azepin

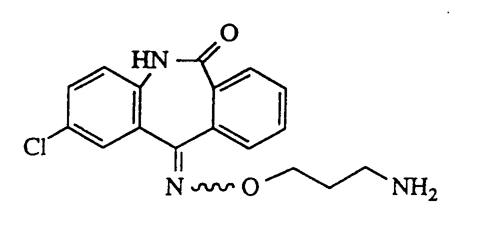

300 mg (1,1 mmol) (E/Z)-2-Chlor-11-hydroxyimino-6-oxo-5,6-dihydro-11H-dibenz[b,e]azepin in 2,2 ml abs. THF werden mit 36,3 mg (1,1 mmol) NaH (80% Suspension in Öl) versetzt und 30 min unter Rückfluß gehalten. Anschließend versetzt man mit 271 mg (1,2 mmol) N-(3-Chlorpropyl)phthalimid und hält 12 h unter Rückfluß. Nach Abkühlen wird filtriert, das Filtrat eingeengt und an Kieselgel mit Methylenchlorid / Essigester 10:1 gereinigt. Man erhält 58 mg (E/Z)-2-Chlor-11-(3-phthalimidopropyloxyimino)-6-oxo-5,6-dihydro-11H-dibenz[b,e]azepin, die in 0,3 ml Methylenchlorid gelöst werden. Nach Zugabe von 10 µl Hydrazinhydrat läßt man 48 h bei Raumtemperatur rühren und dampft dann ein. Der Rückstand wird in wenig Methanol gelöst und zwischen Methylenchlorid und 1 N Salzsäure verteilt. Die wäßrige Phase wird mit 1 N Natronlauge alkalisch gestellt und 3 mal mit Methylenchlorid extrahiert. Die organischen Phasen werden vereinigt, über MgSO₄ getrocknet und eingeengt. Man erhält 30 mg des 3-Aminopropylderivates als farblosen Schaum.
¹H-NMR (CD₃OD) δ = 1,72 (m, 2H); 2,70 (m, 2H); 4,28 (m, 2H); 7,10 und 7,14 (2d, J = 9Hz, 1H); 7,30 (m, 1H); 7,45-7,65 (m, 4H); 7,95 (d, J = 9 Hz, 1H).

In Analogie zur Vorschrift des Beispiels 1 werden die in Tabelle 1 aufgeführten Beispiele hergestellt:

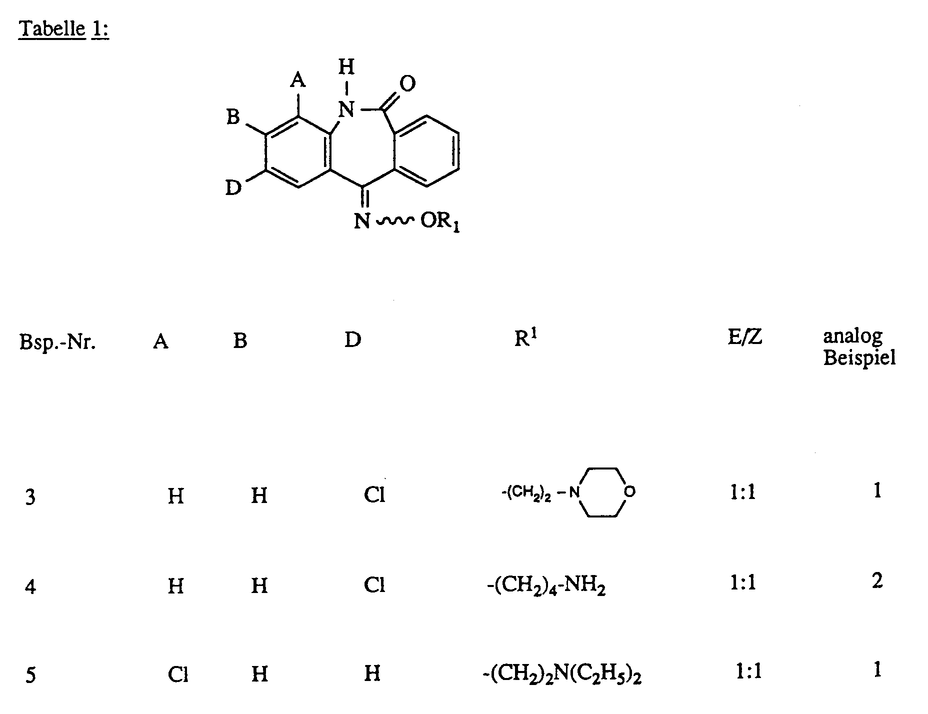

## Patentansprüche

1. Verwendung von Aminoalkyl-substituierten 5,6-Dihydro-dibenz[b,e]azepin-6,11-dion-11-oximen der allgemeinen Formel (I) 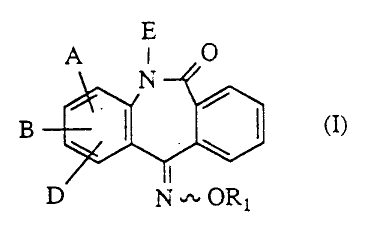 in welcher
A, B und D gleich oder verschieden sind und für Wasserstoff, Amino, Nitro, Halogen, Cyano, Hydroxy, Trifluormethyl, Trifluormethoxy oder für geradkettiges oder verzweigtes Alkyl oder Alkoxy mit jeweils bis zu 8 Kohlenstoffatomen stehen,
E für Wasserstoff oder für geradkettiges oder verzweigtes Alkyl mit bis zu 6 Kohlenstoffatomen steht,
R¹ für geradkettiges oder verzweigtes Alkyl oder Alkenyl mit jeweils bis zu 10 Kohlenstoffatomen steht, die stets durch eine Gruppe der Formel -NR²R³ substituiert sind,
worin
R² und R³ gleich oder verschieden sind und Wasserstoff, geradkettiges oder verzweigtes Alkyl mit bis zu 8 Kohlenstoffatomen oder Phenyl bedeuten, oder
R² und R³ gemeinsam mit dem Stickstoffatom einen 5- bis 7-gliedrigen, gesättigten oder ungesättigten heterocyclischen Ring mit bis zu 2 weiteren Heteroatomen aus der Reihe S, N oder O bilden,
gegebenenfalls in einer isomeren Form und deren physiologisch unbedenklichen Salze zur Herstellung von Arzneimitteln zur Therapie von retroviralen Infektionen.

2. Verwendung von Verbindungen der allgemeinen Formel (I) gemäß Anspruch 1, in welcher
A, B und D gleich oder verschieden sind und für Wasserstoff, Fluor, Chlor, Hydroxy, Trifluormethyl, Trifluormethoxy oder für geradkettiges oder verzweigtes Alkyl oder Alkoxy mit jeweils bis zu 6 Kohlenstoffatomen stehen,
E für Wasserstoff oder für geradkettiges oder verzweigtes Alkyl mit bis zu 4 Kohlenstoffatomen steht,
R¹ für geradkettiges oder verzweigtes Alkyl oder Alkenyl mit jeweils bis zu 8 Kohlenstoffatomen steht, die stets durch eine Gruppe der Formel -NR²R³ substituiert sind,
worin
R² und R³ gleich oder verschieden sind und Wasserstoff oder geradkettiges oder verzweigtes Alkyl mit bis zu 6 Kohlenstoffatomen bedeuten,
oder
R² und R³ gemeinsam mit dem Stickstoffatom einen Morpholin- oder Piperazinring bilden,
gegebenenfalls in einer isomeren Form und deren physiologisch unbedenklichen Salze zur Herstellung von Arzneimitteln zur Therapie von retroviralen Infektionen

3. Verwendung von Verbindungen der allgemeinen Formel (I) gemaß Anspruch 1, in welcher
A, B und D gleich oder verschieden sind und für Wasserstoff, Fluor, Chlor oder für geradkettiges oder verzweigtes Alkyl oder Alkoxy mit jeweils bis zu 4 Kohlenstoffatomen stehen,
E für Wasserstoff, Methyl oder Ethyl steht,
R¹ für geradkettiges oder verzweigtes Alkyl oder Alkenyl mit jeweils bis zu 6 Kohlenstoffatomen steht, die stets durch eine Gruppe der Formel -NR²R³ substituiert sind,
worin
R² und R³ gleich oder verschieden sind und Wasserstoff oder geradkettiges oder verzweigtes Alkyl mit bis zu 4 Kohlenstoffatomen bedeuten,
oder
R² und R³ gemeinsam mit dem Stickstoffatom einen Morpholinring bilden,
gegebenenfalls in einer isomeren Form und deren physiologisch unbedenklichen Salze zur Herstellung von Arzneimitteln zur Therapie von retroviralen Infektionen.

## Claims

1. Use of aminoalkyl-substituted 5,6-dihydro-dibenz[b,e]azepine-6,11-dione-11-oximes of the general formula (I) 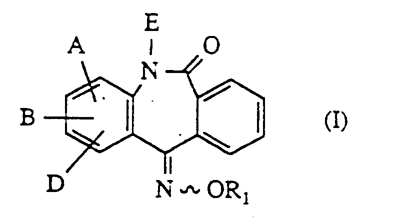 in which
A, B and D are identical or different and represent hydrogen, amino, nitro, halogen, cyano, hydroxyl, trifluoromethyl, trifluoromethoxy or straight-chain or branched alkyl or alkoxy each having up to 8 carbon atoms,
E represents hydrogen or straight-chain or branched alkyl having up to 6 carbon atoms,
R¹ represents straight-chain or branched alkyl or alkenyl each having up to 10 carbon atoms, each of which is always substituted by a group of the formula -NR²R³,
in which
R² and R³ are identical or different and denote hydrogen, straight-chain or branched alkyl having up to 8 carbon atoms or phenyl, or
R² and R³, together with the nitrogen atom, denote a 5- to 7-membered, saturated or unsaturated heterocyclic ring having up to 2 further hetero atoms from the series comprising S, N and O,
if appropriate in an isomeric form, and their physiologically acceptable salts for the production of medicaments for the treatment of retroviral infections.

2. Use if compounds of the general formula (I) according to Claim 1, in which
A, B and D are identical or different and represent hydrogen, fluorine, chlorine, hydroxyl, trifluoromethyl, trifluoromethoxy or straight-chain or branched alkyl or alkoxy each having up to 6 carbon atoms,
E represents hydrogen or straight-chain or branched alkyl having up to 4 carbon atoms,
R¹ represents straight-chain or branched alkyl or alkenyl each having up to 8 carbon atoms, each of which is always substituted by a group of the formula -NR²R³,
in which
R² and R³ are identical or different and denote hydrogen or straight-chain or branched alkyl having up to 6 carbon atoms,
R² and R³, together with the nitrogen atom, form a morpholine or piperazine ring,
if appropriate in an isomeric form, and their physiologically acceptable salts for the production of medicaments for the treatment of retroviral infections.

3. Use of compounds of the general formula (I) according to Claim 1, in which
A, B and D are identical or different and represent hydrogen, fluorine, chlorine or straight-chain or branched alkyl or alkoxy each having up to 4 carbon atoms,
E represents hydrogen, methyl or ethyl,
R¹ represents straight-chain or branched alkyl or alkenyl each having up to 6 carbon atoms, each of which is always substituted by a group of the formula -NR²R³,
in which
R² and R³ are identical or different and denote hydrogen or straight-chain or branched alkyl having up to 4 carbon atoms,
or
R² and R³, together with the nitrogen atom, form a morpholine ring,
if appropriate in an isomeric form, and their physiologically acceptable salts for the production of medicaments for the treatment of retroviral infections.

## Revendications

1. Utilisation de 5,6-dihydro-dibenzo[b,e]azépine-6,11-dione-11-oximes à substituant aminoalkyle, de formule générale (I) 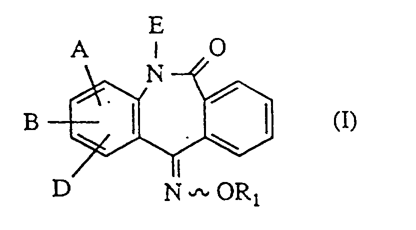 dans laquelle
A, B et D sont identiques ou différents et représentent de l'hydrogène, un groupe amino, nitro, halogéno, cyano, hydroxy, trifluorométhyle, trifluorométhoxy ou un groupe alkyle ou alkoxy linéaire ou ramifié ayant chacun jusqu'à 8 atomes de carbone,
E représente de l'hydrogène ou un groupe alkyle linéaire ou ramifié ayant jusqu'à 6 atomes de carbone,
R¹ est un groupe alkyle ou un groupe alcényle linéaire ou ramifié ayant chacun jusqu'à 10 atomes de carbone, ces groupes étant toujours substitués par un groupe de formule -NR²R³,
où
R² et R³ sont identiques ou différents et représentent de l'hydrogène, un groupe alkyle linéaire ou ramifié ayant jusqu'à 8 atomes de carbone ou un groupe phényle, ou bien
R² et R³ forment, conjointement avec l'atome d'azote, un noyau hétérocyclique pentagonal à heptagonal saturé ou non saturé ayant jusqu'à deux autres hétéroatomes de la série S, N ou O,
le cas échéant sous une forme isomère, et de leurs sels acceptables du point de vue physiologique, pour la préparation de médicaments destinés au traitement d'infections rétrovirales.

2. Utilisation de composés de formule générale (I) suivant la revendication 1, dans laquelle
A, B et D sont identiques ou différents et représentent de l'hydrogène, du fluor, du chlore, un groupe hydroxy, trifluorométhyle, trifluorométhoxy ou un groupe alkyle ou un groupe alkoxy linéaire ou ramifié ayant chacun jusqu'à 6 atomes de carbone,
E est de l'hydrogène ou un groupe alkyle linéaire ou ramifié ayant jusqu'à 4 atomes de carbone,
R¹ est un groupe alkyle ou un groupe alcényle linéaire ou ramifié ayant chacun jusqu'à 8 atomes de carbone, ces groupes étant toujours substitués par un groupe de formule -NR²R³,
où
R² et R³ sont identiques ou différents et représentent de l'hydrogène ou un groupe alkyle linéaire ou ramifié ayant jusqu'à 6 atomes de carbone, ou bien
R² et R³ forment, conjointement avec l'atome d'azote, un noyau de morpholine ou de pipérazine,
le cas échéant sous une forme isomère, et de leurs sels acceptables du point de vue physiologique, pour la préparation de médicaments destinés au traitement d'infections rétrovirales.

3. Utilisation de composés de formule générale (I) suivant la revendication 1, dans laquelle
A, B et D sont identiques ou différents et représentent de l'hydrogène, du fluor, du chlore ou un groupe alkyle ou un groupe alkoxy linéaire ou ramifié ayant chacun jusqu'à 4 atomes de carbone,
E est de l'hydrogène, un groupe méthyle ou éthyle,
R¹ est un groupe alkyle ou un groupe alcényle linéaire ou ramifié ayant chacun jusqu'à 6 atomes de carbone, ces groupes étant toujours substitués par un groupe de formule -NR²R³,
où
R² et R³ sont identiques ou différents et représentent de l'hydrogène ou un groupe alkyle linéaire ou ramifié ayant jusqu'à 4 atomes de carbone, ou bien
R² et R³ forment, conjointement avec l'atome d'azote, un noyau de morpholine,
le cas échéant sous une forme isomère, et de leurs sels acceptables du point de vue physiologique, pour la préparation de médicaments destinés au traitement d'infections rétrovirales.
